(19) Europäisches Patentamt

European Patent Office

Office européen des brevets

(11) **EP 1 142 574 A1**

(12) **EUROPEAN PATENT APPLICATION**

(43) Date of publication:
**10.10.2001 Bulletin 2001/41**

(51) Int Cl.⁷: **A61K 31/43**, A61P 31/04
// (A61K31/43, 31:42)

(21) Application number: **01201884.2**

(22) Date of filing: **10.02.1998**

(84) Designated Contracting States:
**AT BE CH DE DK ES FI FR GB GR IE IT LI LU MC NL PT SE**
Designated Extension States:
**RO SI**

(30) Priority: **14.02.1997 GB 9703099**
**14.02.1997 GB 9703100**
**14.02.1997 GB 9703101**

(62) Document number(s) of the earlier application(s) in accordance with Art. 76 EPC:
**98912303.9 / 0 975 342**

(72) Inventors:
• Mention, Jacky A.,
SmithKline Beecham Lab. Pharma.
53101 Mayenne Cedex (FR)
• Sanroma Bordallo, J.,
SmithKl. Beecham S.A. Pharm.
45007 Toledo (ES)
• Storm, Kevin,
GlaxoSmithKline Pharmaceuticals
Bristol, TN 37620 (US)

(71) Applicants:
• **SMITHKLINE BEECHAM**
**LABORATOIRES PHARMACEUTIQUES**
**92731 Nanterre Cédex (FR)**
• **Smithkline Beecham S.A.**
**28034 Madrid (ES)**
• **SmithKline Beecham Corporation**
**Philadelphia, PA 19103 (US)**

(74) Representative:
**Connell, Anthony Christopher et al**
**SmithKline Beecham plc**
**Corporate Intellectual Property,**
**Two New Horizons Court**
**Brentford, Middlesex TW8 9EP (GB)**

Remarks:
This application was filed on 18 - 05 - 2001 as a divisional application to the application mentioned under INID code 62.

(54) **Pharmaceutical formulations comprising amoxycillin and clavulanate**

(57) Novel co-amoxiclav formulations are described comprising amoxycillin and potassium clavulanate in a ratio of 8:1, to provide a unit dosage of 1000/125mg.

EP 1 142 574 A1

**Description**

The present invention relates to novel formulations comprising amoxycillin and clavulanate (co-amoxiclav), to processes for their preparation and their use in therapy.

The product co-amoxiclav is marketed by SmithKline Beecham as Augmentin for treating bacterial infections. It comprises a combination of the β-lactarn antibacterial agent amoxycillin (present as the trihydrate) and the β lactamase inhibitor clavulanic acid (present as the potassium salt). Various formulations, for instance tablets, capsules, powders (for reconstitution into aqueous syrups) and sachets containing free flowing granules, are provided, containing different ratios of amoxycillin and clavulanic acid. Thus, tablets and powders (for reconstitution into aqueous syrups) are available comprising amoxycillin and clavulanic acid in ratios of 2:1, 4:1 and 7:1, whilst sachets are available with the same ratios, as well as 8:1 (Augmentin, SmithKline Beecham, France, 'poudre pour suspension buvable a 100mg/ml nourisson', comprising 100mg amoxycillin and 12.5mg clavulanate). This leads to a complexity in the manufacturing process as each product with a different ratio is prepared separately, by blending together the different amounts of amoxycillin and clavulanate at an early stage of the process.

In addition, clavulanate has been blended with a diluent, conventionally in a 1:1 ratio, for safer storage and transportation. This has been silica gel (for instance the product Syloid AL-1) for non-tablet formulations and microcrystalline cellulose (for instance the product Avicel) for tablet formulations, adding to further complexity in the manufacturing process.

There therefore remains the need to devise more efficient manufacturing processes, to reduce complexity and improve the economics of the process. It has now been found that this can be achieved by the use of a common intermediate granulate comprising a fixed ratio of amoxycillin and clavulanate, with the differing ratios in the final formulation then being achieved by adding in appropriate amounts of amoxycillin at a later stage in the process. Formulations having granulates comprising amoxycillin and clavulanate are described in GB 2 005 538-A (Beecham Group), WO 95/28927 (SmithKline Beecham) and WO 95/25516 (SmithKline Beecham). There is however no suggestion of combining granulates of amoxycillin and clavulanate with granulates of amoxycillin.

As the absolute amount of drug substance increases, the tablet size also increases, making the tablets less attractive to swallow. Tablets further comprise excipients such as disintegrants, diluents, lubricants which are necessary to allow the tablet to be manufactured and to enhance the pharmacokinetic performance of the tablets, once taken. Such excipients further add to the gross weight of a tablet. Furthermore, the conventional practice of formulating tablets comprising potassium clavulanate from a 1:1 blend of potassium clavulanate and a diluent, referred to above, further adds to the weight and size of a tablet. Thus, currently available (coated) tablets comprising 500/125 and 875/125 mg amoxycillin plus clavulanate (expressed as the weight of the corresponding free acids) have weights of 1050 and 1482mg, respectively, of which the drug substances account for about 70% and 81% respectively, by weight. Prototype formulations disclosed in earlier patent applications suggest 500/125mg tablets weighing 872mg (uncoated) (Example 1, GB 2 005 538-A), 950mg (uncoated), 1050mg (coated) (Example 14 &15, WO 92/19227) and 875/125mg tablets weighing 1350mg (uncoated) and 1450mg (coated) (Example 14 &15, WO 92/19227). There is therefore a need to provide tablet formulations having a smaller size for a given amount of drug substance.

A new co-amoxiclav dosage regimen of 1000/125 mg twice a day (bd) has generated a need for an appropriate tablet formulation(s). This unit dose has not so far been provided as a tablet formulation as merely increasing the size of the exisiting 875/125mg tablet was considered to give a tablet which would be unacceptably large for the patient to swallow..

Accordingly, in a first aspect, the present invention provides a pharmaceutical formulation comprising amoxycillin and clavulanate in a ratio of n: 1 where n is a number from 1 to 16 which comprises:

    a first set of granulates comprising amoxycillin and clavulanate in a ratio from m: to 1:1 where m is a number less than n; and
    a second set of granulates comprising amoxycillin and no clavulanate;
    in a ratio between the first and second sets of granulates to give an overall ratio between amoxycillin and clavulanate of n: 1.

Preferably, n is 2, 4, 6, 7, 8, or 14.

Preferably, m is a number from 1 to 5, more preferably, 1, 2 or 4.

Preferably, the first set of granulates comprise amoxycillin and clavulanate in a ratio of 1:1, 2:1 or 4:1, preferably 2:1.

In a further aspect, the present invention provides for a range of pharmaceutical formulations comprising different ratios of amoxycillin and clavulanate from 2:1 to 14:1, for instance selected from 2:1, 4:1, 6:1, 7:1, 8:1 and 14:1, which is prepared from a first set of granulates having a fixed ratio of amoxycillin and clavulanate, for instance 1:1 or 2:1, preferably 2:1, and a second set of granulates comprising amoxycillin, by combining different relative proportions of the two sets of granulates.

Thus, for instance, a range comprising formulations having ratios of 4:1, 7:1 and 8:1 may be prepared by combining granulates comprising amoxycillin and clavulanate in the ratio 2:1 with different relative amounts of granulates comprising amoxycillin, for instance in the ratios 3:2, 3:5 and 3:6 respectively.

The terms "amoxycillin" and "clavulanate" used herein, and unless otherwise specified, include both the free parent acids and derivatives such as salts thereof.

Weights and ratios are expressed in terms of the weight of parent compound amoxycillin or clavulanic acid, this terminology being used throughout this description unless otherwise stated.

Suitable derivatives of amoxycillin are amoxycillin trihydrate, anhydrous amoxycillin and alkali metal salts of amoxycillin such as sodium amoxycillin. Preferably, the equilibrium relative humidity (ERH) of the amoxycillin trihydrate used as a raw material for granulate production is carefully controlled by appropriate drying so that it doe not compromise other aspects of the formulation. Preferably, the ERH is less than 50%, more preferably less than 30%, most preferably from 10 to 20%.

Suitable derivatives of clavulanic acid are alkali metal salts of clavulanic acid such as potassium clavulanate.

Preferably, formulations of this invention comprise amoxycillin trihydrate and potassium clavulanate, this combination having met with regulatory approval, and being particularly advantageous.

Suitable granulates for use in formulations of the present invention have been previously described in WO 92/19277 (SmithKline Beecham) and GB 2 005 538-A (Beecham Group). Suitable granulates may comprise in addition to amoxycillin and, if present, clavulanate, excipients conventionally used in such granulates, for instance an intragranular disintegrant, intragranular lubricant and intragranular diluent.

Suitable intra-granular disintegrants include starches, such as maize starch and rice starch, crospovidone (cross-linked N-vinyl-2-pyrrolidinone; CLPVP), sodium starch glycollate, croscarmellose sodium and formaldehyde - casein, or combinations thereof. Preferred intra-granular disintegrants include sodium starch glycollate and CLPVP, in particular CLPVP, for example the product marketed under the trade names Polyplasdone XL and Polyplasdone XL-10. Preferably, the intragranular disintegrant is present in from 0.1 to 10%, preferably from 1.0 to 8.0%, more preferably from 1.25 to 3.5% by weight of the granulate.

Suitable intragranular lubricants are those conventional to the art, such as long-chain fatty acids, such as stearic acid, or salts thereof, in particular Group II metal salts, such as of magnesium or calcium. A preferred lubricant is magnesium stearate. Preferably, a lubricant is used in as low a proportion for instance form 0 to 1%, preferably from 0 to 0.5%, more preferably from 0 to 0.35%. Preferably, if the granulate is formed from ingredients which have been compacted using a roller compactor, it is possible to use no lubricant at all.

Preferably, when the granulate contains potassium clavulanate, granulates also include an intragranular diluent such as silica gel (which may also act as an intragranular dessicant), for instance the product Syloid AL-1, or microcrystalline cellulose (which may also act as a compression aid), for instance the product Avicel. Preferably, the intragranular diluent, if present, is silica gel on account of its superior dessicating power. Conventionally, a diluent is included in a weight ratio of about 1:1 with respect to the weight of potassium clavulanate, the granulate being prepared from a 1:1 blend of potassium clavulanate and silica gel or microcrystalline cellulose. It has however also been found possible to reduce the relative to amount of intragranular diluent, in particular silica gel, to a much lower level, and even avoid the use thereof. Accordingly, in a further aspect, the present invention provides for a granulate comprising amoxycillin and clavulanate as hereinbefore defined and an intragranular diluent present in from 0 to 100%, preferably from 0 to 50%, more preferably 5 to 25%, most preferably 5 to 15%, typically about 10% by weight of potassium clavulanate. Preferably, the dessicant diluent is included in a proportion of about 0.5-5% by weight of the amoxycillin plus clavulanate, more preferably from 1 to 3%. Such granulates allow the preparation of final formulations, in particular tablets, having a reduced overall weight and size.

Typically the proportion of amoxycillin and potassium clavulanate, if present, in a granulate is from 90 to 99.9wt %, preferably from 92 to 99wt %, for instance from 95 to 99wt %, such as 96.5 to 98.75% of the weight of the granulate. When the granulate contains a diluent, this may comprise up to 30wt % of the granulate, preferably, up to 25wt%, more preferably up to 10wt%, preferably up to 5wt%. When the granulate contains a diluent, the granulate will contain a correspondingly lower proportion amoxycillin or amoxycillin plus clavulanate, for example from 70 to 99.9wt % of the granulate, preferably from 85%, more preferably from 90%, typically from 93 to 97wt%.

Preferred amoxycillin granulates comprise from 95 to 99wt%, preferably from 96 to 98wt%, more preferably from 96.5 to 97.5wt% amoxycillin trihydrate and from 1 to 5wt%, preferably 2 to 4wt%, more preferably from 2.5 to 3.5wt% of starch glycollate or CLPVP. Most preferred granulates consist essentially of about 97wt% amoxycillin trihydrate and 3wt% CLPVP.

Preferred amoxycillin and potassium clavulanate granulates comprise a 2:1 ratio of amoxycillin to clavulanate; silica gel present in from 5 to 15wt% of potassium clavulanate, typically about 10wt%; and CLPVP present in from 0.5 to 5wt% of amoxycillin trihydrate, preferably 1 to 4wt%, typically about 3%.

Preferably the particles of amoxycillin and clavulanate, if present, in the granulates are in the size range 1

μm to 300μm, especially 10μm to 200μm. A typical suitable size distribution of the amoxycillin/clavulanic acid particles is : >200μ, 5% or less; 200-100μ, 5-15%; 100-50μ, 7.5-15%; <50μ, 70% or more.

Granulates according to the present invention may be presented in a variety of finished formulations, including, for instance, tablets, for example, swallow tablets, dispersible tablets and chewable, optionally effervescent, tablets; in capsules; aqueous syrups and sachets. These may be prepared by combining the granulates with additional, extra-granular, excipients conventionally used in such formulations and further processing into finished formulations.

Tablets of the present invention may include an extra-granular disintegrant, for instance maize-starch and rice starch, CLPVP, sodium starch glycollate, croscarmellose sodium, microcrystalline or microfine cellulose, low-substituted hydroxypropylcellulose (i.e. cellulose partially substituted with 2-hydroxypropyl groups, e.g. less than 25% substituted, preferably 7-16% substituted), cross-linked sodium carboxymethylcellulose, swellable ion exchange resins, formaldehyde-casein, or alginates. A preferred extra-granular disintegrant is low-substituted hydroxypropylcellulose, for instance the product L-HPC LH-11 (from Shinetsu). This also has useful binding properties, allowing the preparation of tablets with good hardness for a relatively lower proportion of ingredient. Other useful extra-granular disintegrants include CLPVP and sodium starch glycollate.

The proportion of extra-granular disintegrant to total tablet weight may vary between broad limits, for example 0.1-25 weight %. For example, low-substituted hydroxypropylcellulose, CLPVP or sodium starch glycollate may preferably be used in a proportion 0.1-15.0 weight %, preferably 1.0 - 10.0 weight %, preferably 3.0-8.0 weight % of the total tablet weight. If cellulose or a combination containing cellulose is used, e.g. as described above containing around 80-90% by weight of cellulose, the extra-granular disintegrant may comprise 1-20 weight %. Dispersible tablets will tend to comprise a relatively higher proportion of extra-granular disintegrant, to aid the dissolution process.

Tablets of the present invention may also include an extra-granular lubricant, for instance a long-chain fatty acid, such as stearic acid, or salts thereof, in particular Group II metal salt thereof, such as a magnesium or calcium salt, preferably magnesium stearate. Preferably, the extra-granular lubricant is used in from 0.1 to 2%, more preferably from 0.2 to 0.5, typically, 0.3 to 0.4 weight% of the tablet.

Formulations of the present invention may also include an extra-granular dessicant such as silica gel. This may be present in an amount up to 5% by weight of the formulation, preferably, up to 2% of a tablet formulation. Careful control of the Equilibrium Relative Humidity of the amoxycillin trihydrate used for the granulates allows the use of a relatively lesser proportion of an extra-granular dessicant.

Tablets may also include other conventional excipients, typically present up to about 10% of the total tablet weight, for instance flavoring agents, for example flavorings such as menthol, peppermint, vanilla or fruit flavorings, flavoring agents typically being present up to around 0.5-5% by weight of the whole tablet and sweeteners, e.g. aspartame, present of up to around 15mg per unit dose. Excipients may also include colouring agents, preservatives, suspending aids and fillers such as silicon dioxide, microcrystalline cellulose, dicalcium phosphate, lactose, sorbitol, calcium carbonate or magnesium carbonate. Such excipients are preferably mixed with the extra-granular disintegrant and lubricant (if present). The materials present in the tablets should have low free moisture content and preferably be pre-dried.

Tablets of the present invention may also contain an effervescent couple of known type, e.g. a solid acid and an alkali metal carbonate or bicarbonate which generates carbon dioxide on contact with water to assist in disintegration of the tablet, for instance monosodium citrate (56.04% w/w) and sodium bicarbonate(43.96% w/w). Preferably, the couple is provided as granules prepared from anhydrous powdered monosodium citrate and powdered sodium bicarbonate and compacted together by roller compaction, according to the process described in WO 97/02014 (SmithKline Beecham Laboratoires Pharmaceutiques).

The tablets may be film coated in a conventional manner, e.g. for cosmetic, palatability or production purposes. Suitable coatings include hydroxypropylcellulose, acrylate and/or methacrylate co-polymers such as the products available under the trade mark Eudragit, resins etc. Alternatively the coating may be an enteric coating, e.g. which is insoluble in acidic gastric juice but soluble in alkaline digestive juice. Such a coating enables the tablet to pass through the stomach into the duodenum, from where it is absorbed. Suitable enteric coatings include cellulose acetate phthalate.

Preferably, a film coating is applied by aqueous film coating techniques, thereby avoiding the need for organic solvents. Suitable polymers for use in such techniques include hydroxypropylcellulose, hydroxypropylmethyl cellulose, ethylcellulose (for example ethylcellulose in a latex composition as supplied by the FMC Corporation as "Aqua-Coat" (trade mark)), methylhydroxyethylcellulose, polyvinylpyrrolidone ("PVP", e.g. as supplied under the name Povidone (trade mark), sodium carboxymethylcellulose and acrylate polymers (e.g. the known methacrylic acid esters supplied under the trade name "Eudragit" (trade mark)).

A preferred polymer is hydroxypropylmethylcellulose ("HPMC") preferably in combination with a polyethylene glycol ("PEG"). PEG's of low molecular weight (200 to 600 series) are liquid at room temperature and find use as plasticisers. PEG's with high molecular weights (900 to 8000) are waxy solids at room temperature and are used in combination with low molecular weight PEG's and with other polymers such as HPMC to modify film properties and to contribute to tablet sheen.

A preferred polymer which can be applied by aqueous film coating techniques is one or more hydroxypropylmethyl celluloses combined with one or more PEG's. HPMC polymers have the advantages of solubility in physiological fluids as well as water, non-interference with tablet disintegration, dissolubility or drug availability, formation of a flexible film, freedom from objectionable taste or odour, stability to heat, light, air, moisture, compatibility to stabilisers, colourants opacifiers, and gloss. The hydroxypropylmethylcellulose functions as a film former, and the polyethylene glycol functions as a plasticiser. The hydroxypropylmethyl cellulose: polyethylene glycol ratio in the film coating is preferably between 7.5 : 1 to 5.5 : 1, e.g. around 6.5 : 1 ±10%. Preferably the hydroxypropylmethyl cellulose is applied in the form of a mixture of hydroxypropylmethyl cellulose 6 cps and 15 cps, in a ratio of around 2:1 to 4:1 e.g. around 3:1±10%. Preferably the polyethylene glycol is applied in the form of a mixture of polyethylene glycol 4000 and 6000 in a ratio between around 1:2 to 2:1, e.g. around 1:1. The film coat may also preferably include an opacifier, for example titanium dioxide (white). Preferably the opacifier may be present in around a 1:1 ± 10% proportion with the hydroxypropylmethyl cellulose in the film coat.

The materials of the film coat are preferably applied by an aqueous film coating process, as application in this way form a film of a nature which also appears to contribute to the improved consistency in bioavailability. A suitable solids loading for the aqueous film coat is around 10-30% w/v, typically 10-20%, e.g. 15% + 2%.

Preferably the film coating is applied so as to deposit a weight of dried film materials corresponding to between 0.5 and 5%, preferably 1 and 4%, more preferably 1.5 to 3.5% of the total coated tablet weight.

Preferably the proportion of amoxycillin and clavulanate in tablets of the present invention is 60-98% by weight of the total tablet (calculated as the weight of the particular form used, for instance the weight of amoxycillin trihydrate or potassium clavulanate).

Preferably, tablets according to the present invention are provided in convenient unit dosage forms, for instance comprising nominally 125/62.5, 250/62.5, 250/125, 500/62.5, 500/125, 875/125 and 1000/125mg amoxycillin/ clavulanate. The tablets may be dispersed in water prior to ingestion, or may alternatively be chewed or swallowed whole.

The skilled man will readily appreciate that, in tablets of this invention, the granulates may be in a crushed state resulting from the compaction of the tablet, and consequently may not have discrete boundaries, or may be subdivided or broken up into smaller granulates. The invention is intended to include.tablets having such a structure containing crushed granulates. Preferably the size of the granulates is in the range 100μm to 2mm, preferably around 1mm ± 0.25mm, maximum dimension.

Preferably the tablets are packaged in a container that inhibits the ingress of atmospheric moisture, e.g. blister packs or tightly closeable bottles etc. as conventional in the art. Preferably bottles also include a desiccant material to preserve the clavulanare.

By reducing the relative amount of excipients used, in particular intra-granular diluent, as well as minimising the amounts of extra-granular excipients used in a tablet formulation, tablets of reduced weight may be prepared. This is assisted by careful control of the moisture content of the various excipients, as well as amoxycillin trihydrate, to ensure that a larger dessicating capacity is not required. Accordingly, in a further aspect, the present invention provides for a tablet formulation comprising a first set of granulates comprising amoxycillin and clavulanate with a second set of granulates comprising amoxycillin and no clavulanate as hereinbefore defined in which the combined weight of all excipients is less than 20%, preferably less than 18%, more preferably less than 15%, typically from 10 to 12%, of the uncoated core weight of the tablet. This provides tablets which are easier for the patient to swallow. In addition, such reduced weight tablets cost less to produce as less raw materials are used, less material has to be transported around a manufacturing plant and the capacity of the equipment is effectively increased, since less material is processed per tablet.

Preferred reduced weight tablets comprise:

amoxycillin granulates which consist essentially of amoxycillin trihydrate present in about 97wt% and CLPVP present in about 3wt%;
amoxycillin and potassium clavulanate granulates which comprise a 2:1 ratio of amoxycillin to clavulanate; silica gel present in about 10wt% of potassium clavulanate and about 3wt% of CLPVP; and
extra granular excipients including:
silica gel (preferably 0.5 to 2.0%, more preferably about 1.0 to 1.2%); low-substituted hydroxypropylcellulose (preferably 3 to 8%, more preferably 5 to 6%); colloidal silica (preferably 0.1 to 0.5%, more preferably 0.1 to 0.3%); and magnesium stearate (preferably 0.2 to 0.5%, more preferably 0.3 to 0.4%); or
CLPVP (preferably 3 to 10%, more preferably 5 to 8%); and magnesium stearate (preferably 0.2 to 0.5%, more preferably 0.3 to 0.4%).

Preferred 500/62.5mg tablets will have tablet core weights in the range 700 to 800mg, more preferably 720 to 780mg. Preferred 500/125mg tablets will have tablet core weights in the range 800 to 950mg, more preferably 800 to 900mg, most preferably 800 to 850mg.

Preferred 875/125mg tablets will have tablet core weights in the range 1250 to 1375mg, more preferably 1250 to 1325mg. Preferred 1000/125mg tablets will have tablet core weights in the range 1400 to 1550mg, more preferably 1425 to 1475mg.

Especially preferred 500/125mg tablets will have a total weight (core plus coating) of less than 900mg, most preferably less than 850mg. Especially preferred 500/62.5mg tablets will have a total weight (core plus coating) of less than 850mg, most preferably less than 800mg. Especially preferred 875/125mg tablets will have a total weight (core plus coating) of less than 1400mg, most preferably less than 1350mg. Especially preferred 1000/125mg tablets will have a total weight (core plus coating) of less than 1550mg, preferably less than 1500mg.

The skilled person will readily appreciate that the above mentioned tablets may also be produced by a more conventional approach, using a single set of granules comprising the amoxycillin and clavulanate in the final ratio of, for instance, 4:1, 7:1 or 8:1, rather than the two sets of granules hereinbefore described, using intra- and extra-granular excipients in the same proportions. The present invention covers all such tablets.

As hereinbefore described, a 1000/125mg unit dosage in a tablet formulation may be provided as a single, preferably reduced weight, tablet. Preferred reduced weight 1000/125mg tablets comprise amoxycillin and clavulanate present in from 83 to 95%, preferably 85 to 93%, more preferably, 87 to 91% of the core tablet weight and pharmaceutically acceptable excipients present in from 5 to 17%, preferably 7 to 15%, more preferably, 9 to 13% by weight of the core weight of the tablet (excluding any coating). The skilled person will however readily appreciate that the dosage may also be provided using two 500/62.5mg tablets (ratio amox/clav = 8:1). Such tablets will be intrinsically smaller as they contain less drug substance so such tablets may also be based on more conventional formulations, for instance existing 500/125mg formulations where there is not such a need to reduce the weight and size of the tablet. Accordingly, in a further aspect, the present invention provides for a tablet comprising about 500mg amoxycillin and about 62.5mg potassium clavulanate and pharmaceutically acceptable excipients. The skilled person will further appreciate that a 1000/125mg dosage may also be provided by the combination of a 500/125mg amoxycillin/clavulanate tablet and a 500mg amoxycillin tablet, for instance using existing such tablets or adapting one or the other to more readily distinguish between the two, for instance by shape or colour. Patient compliance with such two tablet strategies may be enhanced by providing the tablets in a blister pack presentation, with each blister containing two tablets. The present invention covers all such approaches.

A 1000/125mg unit dosage may also be provided as a chewable, optionally effervescent tablet, whereby the problem of having to swallow whole a large tablet is overcome by the patient chewing the tablet and breaking it down into smaller pieces prior to swallowing. Accordingly, in a further aspect, the present invention provides a chewable, optionally effervescent, tablet comprising 1000/125mg amoxycillin and clavulanate.

Chewable tablets may be prepared by combining granulates as hereinbefore described with extra-granular excipients conventionally used to form a chewable base, for instance, mannitol, sorbitol, dextrose, fructose, or lactose, alone or in combination, preferably present in from 10 to 30% based on the weight of the final tablet, more preferably from 15 to 25%. The tablets may also contain conventional lubricants such as magnesium stearate, sweetening agents such as aspartame or sodium saccharin and flavouring and colouring agents. A disintegrating agent may also be incorporated as an extragranular excipient, to give the patient the option of dispersing the tablet in a small amount of water prior to administration. Suitable disintegrating agents include cellulose-based products such microfine cellulose, microcrystalline cellulose or hydroxypropyl cellulose as well as sodium strach glycollate and CLPVP. The disintegrant is preferably present in from 5 to 30%, more preferably from 5 to 15%, based on the weight of the final tablet.

Preferably, the chewable tablet is also provided with an effervescent couple. Effervescent chewable tablet formulations comprising 250/125 or 250/62.5mg amoxycillin/clavulanate have previously been described in EP 0 396 335 A1 (Beecham Group plc). Suitable such effervescent couples are well known in the art and typically comprise comprise a solid acid and an alkali metal carbonate or bicarbonate which generates carbon dioxide on contact with water, for instance citric acid, monosodium citrate or sodium hydrogen citrate and sodium (bi)carbonate. Other pharmaceutically acceptable acid/alkaline or alkaline earth metal carbonate mixtures may also be used, for instance tartaric, adipic, fumaric or malic acids and sodium, potassium or calcium (bi)carbonates or sodium glycine carbonate. Preferably, the two components are employed in a chemical molecular equivalent basis in the range 4:3 to 1:3, more preferably about 2:3 (acidic:basic component).

Preferred couples comprises citric acid/sodium bicarbonate and monosodium citrate/sodium bicarbonate, in particular monosodium citrate (56.04% w/w) and sodium bicarbonate(43.96% w/w). Preferably, this is provided as granules prepared from anhydrous powdered monosodium citrate and powdered sodium bicarbonate and compacted together by roller compaction, according to the process described in WO 97/02014 (SmithKline Beecham Laboratoires Pharmaceutiques). Suitable grades of anhydrous powdered monosodium citrate comprise particles which are substantially (i.e. about more than 90%) within the range 0 to 500 microns, preferably 355 microns, more preferably 0 to 250 microns. Suitable grades are available from Roche (monosodium citrate anhydrous powder, maximum of 5%w/w with grain size >0.250mm), Boehringer Ingelheim (monosodium zitrate wasserfrei Art-Nr 661 511, minimum of 90%w/w with grain size < 0.150mm), Jungbunzlauer (maximum of 5%w/w with grain size >0.355mm) and Haarmann & Reimer Corp. (maximum of 1% with grain size >0.500mm). Suitable grades of powdered sodium bicarbonate comprise particles which are substantally (i.e. about more than 90%) within the range of 0 to 500 microns, preferably 270 microns,more preferably 0 to 130 microns. Suitable grades of powdered sodium bicarbonate are available from Solvay, for instance the grades 0 to 13 (particle size, by sieving method: >0.16mm max; 15g/kg) and extra-fine (particle size by sieving method: >0.125mm max; 20g/kg).

Preferably, the couple is present in from 5 to 30% of the final tablet weight, more preferably, from 5 to 15%, typically about 10%.

The granulates hereinbefore described may also be used for preparing other pharmaceutical formulations, in addition to tablets. For instance, they may be supplied as a sachet product containing a free-flowing granulated formulation in a suitable unit dose, for reconstituting in water to form an syrup formulation immediately prior to use, for example for administration to small children. Such free-flowing granulated formulation may comprise further excipients such as sweetening agents, thickeners, preservatives such as sodium benzoate and buffers such as sodium citrate.

Preferred sachets comprise:

amoxycillin granulates which consist essentially of amoxycillin trihydrate present in about 97wt% and CLPVP present in about 3wt%;
amoxycillin and potassium clavulanate granulates which comprise a 2:1 ratio of amoxycillin to clavulanate; silica gel present in about 10wt% of potassium clavulanate and
CLPVP present in about 3wt% of amoxycillin trihydrate; and
extra granular excipients including silica gel (preferably 5 to 20%, more preferably about 10 to 15%), and flavour (preferably 2 to 10%, more preferably 3 to 8%).

Such sachets may preferably be provided as 'sugar-free' formulations, comprising, as an artificial sweetening agent such as aspartame, rather than sugar. Preferably, such formulations comprise preferably 1 to 10%, more preferably about 1 to 5% aspartame.

Such sachet formulations are of reduced weight in comparison to existing sachet formulations comprising corresponding weights of amoxycillin and clavulanate and therefore may be provided in smaller sachets, saving on packaging costs.

Further suitable formulations include encapsulated formulations which may optionally include an extra-granular lubricant, which if present is preferably in an amount of less than 0.5% by weight of the granulates, being contained within a pharmaceutical capsule. The pharmaceutical capsule may be an entirely conventional capsule, capable of dissolving in the stomach to release its contents, for example made of gelatine.

The sachet and encapsulated formulations described above preferably contain unit doses of amoxycillin, for instance sachets comprising nominally 125/31.25, 250/31.25, 250/62.5, 500/125, 500/62.5, 875/125 or 1000/125mg of amoxycillin/clavulanate.

Other suitable formulations include formulations for paediatric use which are reconstituted into aqueous suspensions prior to use. Such formulations may comprise further extragranular excipients such as, for example, a dessicating agent, for instance silica gel or dried maltodextrin, thickeners such as xanthan gum, carboxymethylcellulose sodium, silica gel, and hydroxypropylmethyl cellulose, preservatives such as sodium benzoate, sweetening agents such as aspartame, acesulfamate potassium, and sodium saccharin, lubricants such as magesium stearate, glidants such as colloidal silicon dioxide, and flavouring agents such as fruit flavour(s). Such formulations are found to be less powdery and so donot coat the inside of the bottle containing them , thereby enhancing their asethetic attributes. Further more, preferred formulations have a lower bulk so can be provided in a smaller container. Formulations are provided comprising an appropriate quantity of amoxycillin and clavulanate to be reconstituted into aqueous solution, for instance 100/12.5mg/ml in 30ml, 100/12.5mg/ml in 60ml, 125/31.25mg/5ml in 60 to 80ml and 250/62.5mg/5 ml in 60 to 80ml.

The skilled person will readily appreciate that the aforementioned sachet, encapsulated and paediatric formulations may also be produced by a more conventional approach, using a single set of granules comprising the amoxycillin and clavulanate in the final ratio of, for instance, 4:1, 7:1 or 8:1, rather than the two sets of granules hereinbefore described, using intra- and extra-granular excipients in the same proportions. The present invention covers all such formulations.

In a further aspect, the present invention provides for pharmaceutical formulations comprising amoxycillin and clavulanate in a ratio of n:1, as hereinbefore defined, preferably 2:1, 4:1, 7:1 and 8:1, and which comprise granulates consisting essentiallly of amoxycillin and clavulanate, CLPVP (as an intragranular disintegrant) present in from 0.5 to 5wt% of amoxycillin trihydrate, preferably 1 to 4wt%, typically about 3% and silica gel (as an intragranular diluent/dessicant) present in from 5 to 15wt% of potassium clavulanate, typically about 10wt%.

In a further aspect, the present invention provides a process for preparing the formulations of the present invention hereinbefore defined which process comprises blending together a first set of granulates comprising amoxycillin and clavulanate with a second set of granulates comprising amoxycillin and no clavulanate in an appropriate ratio and then further processing to obtain the final desired formulation, for instance, compressing into tablets, filling into sachets, bottles or capsules.

It will be readily appreciated that a range of different formulations with differing ratios of amoxycillin to clavulanate may be readily be prepared by utilising as a common intermediate granulates comprising a given ratio of amoxycillin and clavulanate, for instance 2:1, and then adding in differing relative amounts of granulates comprising amoxycillin alone, to provide the different final ratios.

It will be further appreciated that the use of granulates comprising silica gel and silica gel as an extra-granular excipient, rather than microfine cellulose allows the development of a range of diverse formulation types with fewer different types of excipients, thereby further reducing the complexity of the manufacturing process.

Preferably, the amoxycillin and clavulanate, if present, (drug substance) is milled and sieved to achieve the desired particle size range. Preferably, the intra-granular disintegrant is also milled and sieved to a suitable particle size, for example, in the case of CLPVP, about 30μ, but particle size does not appear to be critical. Prepared drug substance and intra-granular excipients are then blended together in a dry state, and compacted under pressure. This may be by conventional dry compaction means, for example pressing, rolling, slugging extrusion etc, and a suitable pressure for the compaction process is 30-200KN, for instance 70-100, preferably, 80-90kN for amoxycillin granulates and 50-80, preferably, 60-70kN for amoxycillin/clavulanate granulates. The above-described granulate formulations are particularly suited to formation by roller compaction. The use of roller compaction to prepare granules comprising amoxycillin and potassium clavulanate is described in WO 92/19227 and WO 95/28927 (both to SmithKline Beecham).

Potassium clavulanate is known to be highly sensitive to moisture so that it is preferred that the preparation of the formulations of the invention is carried out under conditions of low humidity, for instance less than 30% RH, more preferably less than 20% RH, ideally 7-10% RH.

For further processing into tablets, it may be necessary to mill and sieve the granulates so as to achieve a suitable size fraction of the granulate. Compression into tablets may be carried out in a conventional manner, for instance on a conventional tabletting machine. As an optional further step the tablets may be coated as described above.

Formulations of this invention may be provided for treatment of bacterial infections generally, for example one or more of *inter alia* upper respiratory tract infections, lower respiratory tract infections, genito- urinary tract infections and skin and soft tissue infections.

Accordingly, in a further aspect, the present invention provides for the use of a first set of granulates comprising amoxycillin and clavulanate with a second set of granulates comprising amoxycillin and no clavulanate in the manufacture of a medicament for treating bacterial infections.

The invention will now be described, by way of example only. In these, the weights and % are the actual weights and % of amoxycillin trihydrate and potassium clavulanate, rather than the corresponding free acid equivalents.

In the following examples it is preferred to use amoxycillin trihydrate in which the Equilibrium Relative Humidity is in the range 10-20%, therebey allowing the relative amount of dessicant to be reduced to a minimum level.

**Example 1 - Amoxycillin granulate**

Amoxycillin trihydrate was milled and sieved using an 0.04 or 0.027 inch (1.0-0.7 mm) aperture sieve, and was mixed for 15 minutes in a blender with dried cross-linked polyvinylpyrrolidinone (CLPVP) having a molecular weight of approximately 1 million and a density of 1.22 mg/cm$^3$ (Polyplasdone XL - Trade Mark), the mixture containing 3.4% of CLPVP by weight. The mixture was consolidated using a roller compacter at a controlled pressure of 50KN. The compacted flakes were granulated in a mill, or granulated through a sieve fitted with a 1mm mesh to obtain a suitable size fraction.

**Example 2 - Amoxycillin granulate**

| Amoxycillin trihydrate | 97.087 % |
|---|---|
| CLPVP (Polyplasdone XL) | 2.913 |

Milled amoxycillin trihydrate and crospovidone (moisture <2%, sieved on a 1mm sieve) were separately weighed out and then introduced into a blender (amox first) for blending together, both operations being carried out in a controlled area (t° = 20 +/- 1°C, RH<30%). The resultant blend was discharged into double polyethylene bags in labelled containers and stored for subsequent roller compaction.

A Bepex roller compactor was fed with blend, maintaining a constant level in the feeding hopper, with the load cell set at 15kN and the compactor at a roller strength of 80-90kN. The resultant flakes were calibrated on oscillating granulator fitted with 1mm aperture screen. Uncompacted material which passed through a 1mm sieve was recycled. The granules were weighed and collected in double polyethylene bags and kegs. The roller compaction and calibration was performed in a controlled area (t° = 20 +/-1°C, RH<30%)

### Example 3 - Amoxycillin granulate

Granulates were prepared using a procedure identical to that of example 1, comprising 97.12 weight % amoxycillin trihydrate together with 2.88 weight % sodium starch glycollate (as "Primogel") as intra-granular disintegrant.

### Example 4 - Amoxycillin/clavulanate granulate

Granulates comprising amoxycillin and clavulante in a ratio of 2:1 were prepared using a procedure identical to example 1, comprising:

| | |
|---|---|
| Amoxycillin trihydrate | 46.3% |
| Potassium clavulanate | 24.3 |
| Silica gel (Syloid AL-1) | 24.3 |
| CLPVP (Polyplasdone XL) (dried) | 1.38 |

Potassium clavulanate and silica gel are used as a 1:1 blend (based on the actual weight of potassium clavulanate).

### Example 5 - Amoxycillin/clavulanate granulate

Granulates comprising amoxycillin and clavulanate in a ratio of 2:1 and a lesser proportion of silica gel than in Example 4 were prepared using a procedure identical to example 1, comprising:

| | |
|---|---|
| Amoxycillin trihydrate | 63.3% |
| Potassium clavulanate | 31.7 |
| Silica gel (Syloid AL-1) | 3.1 |
| CLPVP (Polyplasdone XL) (dried) | 1.9 |

Milled amoxycillin, crospovidone (moisture <2%) and silica gel (sieved on 1 mm) and finally a 1:1 blend of amoxycillin trihydrate/potassium clavulante (sieved on 1 mm) were introduced in succession into a blender and blended together in a controlled area (t° = 20 +/- 1°C,RH<30%). The blend was then discharged into double polyethylene bags with desiccant in labelled containers for storage before roller compaction.

A Bepex roller compactor was fed with blend maintaining a constant level in the feeding hopper, with the load cell set at 15kN; and the compactor at a roller strength of 60-70kN. The resultant flakes were calibrated on oscillating granulator fitted with 1mm aperture screen. Uncompacted material which passed through a 1mm sieve was recycled. The granules were weighed and collected in double polyethylene bags and kegs. The roller compaction and calibration was performed in a controlled area (t° = 20 +/- 1°C, RH<30%)

### Example 6 - Reduced weight tablets

A reduced weight tablet comprising 500mg/62.5mg, 500mg/125mg, 875mg/125mg or 1g/125mg amoxycillin/clavulanate was prepared by combining granulates comprising amoxycillin and potassium clavulanate (Example 5) in a 2:1 ratio with granulates comprising amoxycillin (Example 2), to give an overall ratio of 8:1, 4:1, 7:1 or 8:1 respectively with extragranular excipients, in particular low substituted hydroxypropyl cellulose as the extragranular disintegrant, so that the overall composition was as follows:

| | 500/125 mg | 875/125 mg | 1g/125 mg |
|---|---|---|---|
| **Core** | mg | mg | mg |
| Amoxycillin trihydrate (100% of theory; equivalent to 500, 875mg or 1g Amox. 100%) | 573.87 | 1004.272 | 1147.74 |
| Potassium Clavulanate (100% of theory; equivalent to 125mg Clavulanic acid 100%) | 148.93 | 148.930 | 148.930 |
| Silica Gel[1] (intra-granular) | 14.89 | 14.890 | 14.890 |
| CLPVP (intra-granular) | 17.21 | 30.130 | 34.430 |
| Silica Gel[1] (extra-granular) | 8.3 | 14.000 | 16.000 |
| Low substituted hydroxypropyl cellulose[2] | 50.00 | 70.000 | 80.000 |
| Colloidal silica[3] | 1.70 | 2.700 | 3.000 |
| Magnesium stearate | 3.00 | 4.600 | 5.300 |
| Total weight (core) | 817.9 | 1289.520 | 1450.29 |
| **Coating** | | | |
| Hydroxypropyl methylcellulose 5cP | 6.93 | 10.925 | 12.288 |
| Hydroxypropyl methylcellulose 15cP | 2.31 | 3.642 | 4.096 |
| Polyethylene Glycol 4000 | 1.369 | 2.158 | 2.427 |
| Polyethylene Glycol 6000 | 1.369 | 2.158 | 2.427 |
| Titanium dioxide | 9.022 | 14.225 | 15.997 |
| Total weight of coating | 21 | 33.108 | 37.235 |
| | | | |
| Total weight of coated tablet | 838.9 | 1322.628 | 1487.525 |
| | **500/62.5 mg** | **500/62.5 mg** | **500/62.5 mg** |
| **Core** | mg | mg | mg |
| Amoxycillin trihydrate (100% of theory; equivalent to 500, 875mg or 1g Amox. 100%) | 573.87 | 573.87 | 573.87 |
| Potassium Clavulanate (100% of theory; equivalent to 125mg Clavulanic acid 100%) | 74.46 | 74.46 | 74.46 |
| Silica Gel[1] (intra-granular) | 7.45 | 7.45 | 7.45 |
| CLPVP (intra-granular) | 17.21 | 17.21 | 17.21 |
| Silica Gel[1] (extra-granular) | 7.4 | 15.3 | 17.2 |
| Low substituted hydroxypropyl cellulose[2] | 44.5 | 61.3 | 78.4 |
| Colloidal silica[3] | 1.50 | 1.50 | 1.50 |
| Magnesium stearate | 2.7 | 2.8 | 2.8 |
| Total weight (core) | 729.09 | 753.89 | 772.99 |
| Coating (as above) | 19 | 19 | 19 |
| Total weight of coated tablet | 748.09 | 772.89 | 792.99 |

[1] Syloid AL-1

[2]: L-HPC LH-11 or equivalent;

[3]: Aerosil 200 or equivalent.

The manufacture of tablets was carried out in a controlled area (t° = 20 +/- 1°C, RH<30%). A compression mix was prepared by introducing into a blender Amoxycillin granulates; Amoxycillin/Clavulanate 2/1 granulates; silica gel ,L-HPC LH-11 and colloïdal silicon dioxide sieved on 0.5mm. These materials were blended together. Magnesium stearate (sieved on 0.5mm) was then added, followed by further blending. This mix was then compressed using concave oval punches to give tablets of the desired weight. Uncoated tablets were sampled every 5-15 mn during compression run and checked for hardness, thickness, mean weight, uniformity of mass and friability.

The uncoated tablet cores were then film coated with a coating suspension (15w/w% in water) on a suitable coating machine, rotating the cores and spraying the film coating suspension until theoretical quantity of film coat had been applied (WO 95/28927, SmithKline Beecham).

The film coated tablets were then stored in appropriate sealed containers.

For the 500/125mg tablet, the tablet has a total weight of about 817.9mg, including 63mg of extragranular excipients plus a coating of about 21mg. Amoxicillin trihydrate and potassium clavulanate account for about 89.6% of the core weight of the tablet. This may be contrasted with the present 500/125mg tablet which has a core weight of 1050mg of which amoxycilin trihydrate and potassium clavulanate account for about 69.3% and microcrystalline cellulose about 27%. The quantities may be halved to give a 250/62.5mg tablet

In the 875/125 mg, amoxycillin trihydrate and potassium clavulanate account for about 89.43% of the core weight of the tablet. This can be contrasted with the current 875/125mg tablet which has a core weight of 1450mg, of which amoxycillin trihydrate and potassium clavulanate account for about 80% and microcrystalline cellulose about 15.6% (WO 95/28927, SmithKline Beecham).

In the 1g/125mg tablets, amoxicillin trihydrate and potassium clavulanate account for about 89.41% of the core weight of the tablet. The quantities may be halved, to give a 500/62.5mg tablet.

### Example 7 - Reduced weight tablets

A reduced weight tablet comprising 500mg/125mg, 875mg/125mg or 1g/125mg arnoxycillin/clavulanate was prepared by combining granulates comprising amoxycillin and potassium clavulanate (Example 5) in a 2:1 ratio with granulates comprising amoxycillin (Example 1), to give an overall ratio of 4:1, 7:1 or 8:1 respectively with extragranular excipients, in particular CLPVP as the extragranular disintegrant, so that the overall composition was as follows:

| | 500/125 mg | 875/125 mg | 1g/125 mg |
|---|---|---|---|
| **Core** | mg | mg | mg |
| Amoxycillin trihydrate (based on 86.4% assay for amox; equivalent to 500, 875mg or 1g Amox. 100%) | 578.7 | 1012.7 | 1157.4 |
| Potassium Clavulanate (based on 82.5% assay for KCA; equivalent to 125mg Clavulanic acid 100%) | 151.5 | 151.5 | 151.5 |
| Silica Gel[1] (intra-granular) | 15.2 | 15.2 | 15.2 |
| CLPVP (intra-granular) | 17.4 | 30.4 | 34.7 |
| CLPVP (eactra-granular) | 60 | 90 | 100 |
| Magnesium stearate | 3.00 | 4.50 | 5.0 |
| Total weight (core) | 825.8 | 1304.3 | 1463.8 |

[1] Syloid AL-1

A suitable solvent based coating coating for the cores of the above tablets is as follows:

| | |
|---|---|
| Dimethicone 200 | 0.04mg |
| Ethyl cellulose | 3.21 |
| Hydroxypropylmethyl cellulose 15cps | 12.8 |
| Titanium dioxide suspension | 11.34 |
| Diethyl phthalate | 3.78 |
| Total weight of coating | 31.17 |

### Example 8 - 500mg/125mg and 1g/125mg Sachets

Granulates are prepared using procedures identical to Examples 1 and 5. The granulates are then blended in the appropriate ratio with typical sachet excipients to produce a sachet formula comprising:

| | 500/125 mg | 1g/125mg |
|---|---|---|
| Amoxycillin trihydrate 100% of theory (equivalent to 500mg or 1g amoxycillin 100%) | 573.87 mg | 1147.4 |
| Potassium Clavulanate 100% of theory (equivalent to 125mg Clavulanic acid 100%) | 148.93 | 148.93 |

(continued)

|  | 500/125 mg | 1g/125mg |
|---|---|---|
| CLPVP (intra-granular) | 17.21 | 34.42 |
| Silica gel[1] (intra-granular) | 14.89 | 14.89 |
| Silica gel[1] (extra-granular) | 65.00 | 130.00 |
| Aspartame | 15.00 | 30.00 |
| Peach-Lemon-Strawberry flavour | 30.00 | 60.0 |
| Total Weight | 864.90 | 1565.98 |

[1] Syloid AL-1 Sachets comprising 500/62.5mg and 250.31.25mg may be prepared using *pro rata* amounts based on the 1g/125mg formulation.

### Example 9 - 250/62.5, 500/125 and 875/125mg Sachets

Granulates are prepared using procedures identical to Examples 1 and 5. The granulates are then blended in the appropriate ratio with typical sachet excipients to produce a sachet formula comprising:

|  | 250/62.5 mg | 500/125 mg | 875/125 mg |
|---|---|---|---|
| Amoxycillin trihydrate (based on 86.4% assay for amox, equivalent to 250, 500 and 875 mg amoxycillin 100%) | 289.35 | 573.87 mg | 1147.4 |
| Potassium Clavulanate (based on 82.5% assay for K clav, equivalent to 62.5 and 125mg Clavulanic acid 100%) | 75.75 | 148.93 | 148.93 |
| CLPVP (intra-granular) | 8.7 | 17.4 | 30.4 |
| Silica gel[1] (intra-granular) | 7.6 | 15.2 | 15.2 |
| Silica gel[1] (extra-granular) | 167.5 | 335 | 335 |
| Aspartame | 6 | 12.00 | 12.00 |
| Peach-Lemon-Strawberry flavour | 107 | 214 | 214 |
| Microcrystalline cellulose | 213.1 | 426.2 | 229.2 |

[1] Syloid AL-1

### Example 10 - 100mg/12.5mg/ml suspension - 30ml

Granulates are prepared using procedures identical to Examples 1 and 5. The granulates are then blended in the appropriate ratio with typical suspension excipients to produce a sachet formula comprising:

| Amoxycillin trihydrate 100% of theory (equivalent to 3000mg Amoxycillin 100%) | 3443.22 mg |
|---|---|
| Potassium Clavulanate 100% of theory (equivalent to 375mg Clavulanic acid 100%) | 446.79 |
| CLPVP (intra-granular) | 103.29 |
| Silica gel[1] (intra-granular) | 44.68 |
| Silica gel[1] (extra-granular) | 500.00 |
| Xanthan gum | 25.20 |
| Carboxymethylcellulose sodium salt | 250.00 |
| Sodium benzoate | 51.00 |
| Hydrophobic colloidal silica | 15.00 |
| Aspartame | 96.00 |
| Magnesium stearate | 10.00 |
| Strawberry flavour | 150.00 |
| Total weight | 5135.50 mg |

[1] Syloid AL-1

The formulation is made up into 30ml of aqueous solution immediately prior to first use. The same formulation may also be made up into 60ml aqeous solution.

**Example 11 - Chewable effervescent tablet (1g/125mg)**

|  | mg |
|---|---|
| Amoxycillin trihydrate (equivalent to amoxycillin 1000.0) | 1147.74 |
| Potassium clavulanate (equivalent to clavulanic acid 125.0) | 148.93 |
| CLPVP | 34.43 |
| Silica gel[1] (intragranular) | 14.89 |
| Silica gel[1] (extragranular) | 120.00 |
| Monosodium citrate | 156.91 |
| Sodium bicarbonate | 123.09 |
| Microcrystalline cellulose[2] | 280.00 |
| Flavour | 90.00 |
| Aspartame | 40.00 |
| Magnesium stearate | 30.00 |
| Mannitol DC | 614.01 |
| Total weight | 2,800 |

[1] Crospovidone

[2] Syloid AL-1

[3] Avicel PHI 12

**Example 12 - Conventional 500/125mg tablet**

| Core | |
|---|---|
| Amoxycillin trihydrate (equivalent to amoxycillin 500.0) | 578.87 |
| Potassium clavulanate (equivalent to clavulanic acid 125.0) | 148.93 |
| Anhydrous colloidal silica | 10.50 |
| Sodium starch glycollate[1] | 21.00 |
| Magnesium stearate | 7.27 |
| Microcrystalline cellulose | qs 1050 |
| **Coating** | |
| Methylhydroxypropylcellulose | 11.88 |
| Polyethylene glycol 4000 | 1.76 |
| Polyethylene glycol 6000 | 1.76 |
| Titanium dioxide | 11.60 |
| Dimethicone | 0.14 |
| Coating | 27.14 |
| Total | 1077mg |

[1] (Primojel/Explotab)

**Example 13 - 500/125mg tablet**

| Core | |
|---|---|
| Amoxycillin trihydrate (equivalent to amoxycillin 500.0, based on 86% potency) | 581.4 |
| Potassium clavulanate | 76.2 |
| (equivalent to clavulanic acid 62.5, based on 82% potency) | |
| Colloidal silicon dioxide | 8.2 |
| Sodium starch glycollate | 16.4 |
| Microcrystalline cellulose | 129.1 |
| Magnesium stearate | 5.7 |
| Weight | 817.0 |
| **Coating** | |
| as in Example 6 | 24.5 |
| Total | 841.5mg |

Amocycillin, clavulanate source (either as a 1:1 blend of clav:amox or a 1:1 blend of clav and microcrystalline cellulose), most of the microcrystalline cellulose and a portion of the magnesium stearate were blended together. This blend was then roller-compacted and milled to form granules. These granules were blended with the sodium starch glycollate, colloidal silicon dioxide, the remaining microcrystalline cellulose (approx 38.55 of the total amount) and the magnesium stearate, and the blend then compressed into tablet cores. In a final step, the cores were coated with an aqueous suspension of the coating.

The weights and relative proportions of the components of the foregoing examples 1 to 13 may be varied about the figures listed but preferably are within ±10%, more preferably within ±5%, preferably within ±2.5%. Overages of active ingredients may be used if necessary and justified.

It will be appreciated that the the foregoing formulations may also be produced using a single set of granules comprising amoxycillin and clavulanate in the final ratio of 4:1, 7:1, or 8:1 rather than the two sets described, using intra- and extra-granular excipients in the same proportions. The present invention covers all such formulations.

**Annex**

**New Divisional European Patent Application derived from EP Application No. 989//2303.9**

This divisional application has claims to a tablet comprising 500/62.5 mg of amoxycillin/clavulanate (corresponding to claim 15 and claim 17 of the International application), a unit dosage provided by two such tablets (corresponding to claim 13) and a tablet blister containing two such tablets (corresponding to claim 16 and claim 18 of the International application). New claim 4 is a "second use" claim, relating to the dosage regimen for which the tablets of claim 7 were developed. Basis for this is provided at page 2, line 15 and page 15, lines 16 to 19.

In the IPER, under main heading "Group 3", International claims 17 to 19 were considered to be novel but lack inventive step over D1 to D3, in particular D3. The critical features of the tablet defined by the present claims is the amount of amoxycillin (500 mg) and, separately, the amount of clavulanate (62.5 mg), to give a ratio of 8:1, to provide a unit dosage of 1000/125 mg. It is preferred to use two smaller tablets rather than one larger tablet, to aid swallowing and hence compliance.

D3 is a "formulation" application, describing modified release tablets comprising amoxycillin and clavulanate. The skilled man's attention would be drawn to this. The technology is applicable generally to a wide range of formulations comprising amoxycillin and clavulanate and not limited by the individual weights or ratio of amoxycillin and clavulanate. Thus, the highlighted section, at page 3, lines 9 to 19, describes in a general sense, possible amounts of amoxycillin (in a first list) and, separately, possible amounts of clavulanate (in a second list). The skilled man would recognise that these correspond to the weights used in existing formulations, such that the combinations thereby suggested would also reflect existing combinations, rather than new combinations, for instance 125/62.5, 250/62.5, 500/125 and 875/125 mg. Thus, the skilled man is provided with no incentive to make the specific combination of 500/62.5 mg demanded by the present claims. The incentive is provided by the new use developed for these tablets by the present applicants, as claimed in claim 4. This was a 1000/125 mg dosage regimen to supercede the original 500/125 mg regimen, and which has been approved for use in France. This was initially provided by a 1000/125 mg sachet and, more recently, by two 500/62.5 mg tablets (the subject of the present claims).

In the absence of any such motivation from the prior art, the 500/62.5 mg tablet, to provide a 1000/125 mg unit dosage, has an inventive step.

**Claims**

1.   A tablet formulation comprising about 500 mg amoxycillin and about 62.5 mg potassium clavulanate and pharmaceutically acceptable excipients.

2.   A unit dosage of 1000 mg amoxycillin and 125 mg potassium clavulanate which is provided as two tablets each comprising about 500 mg amoxycillin and about 62.5 mg potassium clavulanate.

3.   A tablet blister pack comprising in each blister two of the tablets defined in claim 1.

4.   The use of amoxycillin and clavulanate in the manufacture of a medicament to provide a unit dosage of 1000 mg amoxycillin and 125 mg clavulanate, for treating bacterial infection.

**European Patent**

**Office**

## EUROPEAN SEARCH REPORT

Application Number

EP 01 20 1884

### DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | WO 95 28148 A (SMITHKLINE BEECHAM PLC ;GRIMMETT FRANCIS WALTER (GB); DAVIDSON NIG) 26 October 1995 (1995-10-26) * page 2, line 9 - line 19 * | 1-4 | A61K31/43 A61P31/04 //(A61K31/43, 31:42) |
| X | US 4 537 887 A (ROOKE DAVID J ET AL) 27 August 1985 (1985-08-27) * column 1, line 49 - column 2, line 11 * | 1-4 | |
| X | US 4 140 764 A (HOWARTH THOMAS T) 20 February 1979 (1979-02-20) * column 6, line 32 - column 7, line 2 * | 1-4 | |
| X | US 4 110 165 A (HOWARTH THOMAS TREVOR ET AL) 29 August 1978 (1978-08-29) * column 7, line 61 - column 8, line 35 * | 1-4 | |
| X | WO 96 07408 A (SMITHKLINE BEECHAM PLC ;CROWLEY PATRICK JOHN (GB)) 14 March 1996 (1996-03-14) * page 7, line 31 - page 9, line 33 * | 1-4 | |
| X | WO 94 16696 A (SMITHKLINE BEECHAM PLC ;SMITH GILLIAN MARJORY (GB); THORBURN CHRIS) 4 August 1994 (1994-08-04) * page 7, line 27 - line 33 * | 1-4 | **TECHNICAL FIELDS SEARCHED** (Int.Cl.7) A61K |

The present search report has been drawn up for all claims

| Place of search | Date of completion of the search | Examiner |
|---|---|---|
| THE HAGUE | 15 August 2001 | Leherte, C |

EPO FORM 1503 03.82 (P04C01)

**ANNEX TO THE EUROPEAN SEARCH REPORT
ON EUROPEAN PATENT APPLICATION NO.**

EP 01 20 1884

This annex lists the patent family members relating to the patent documents cited in the above-mentioned European search report.
The members are as contained in the European Patent Office EDP file on
The European Patent Office is in no way liable for these particulars which are merely given for the purpose of information.

15-08-2001

| Patent document cited in search report | | Publication date | Patent family member(s) | | | Publication date |
|---|---|---|---|---|---|---|
| WO 9528148 | A | 26-10-1995 | EP | 0752850 | A | 15-01-1997 |
| | | | JP | 9511994 | T | 02-12-1997 |
| | | | US | 6136345 | A | 24-10-2000 |
| US 4537887 | A | 27-08-1985 | AU | 549321 | B | 23-01-1986 |
| | | | AU | 7568481 | A | 08-04-1982 |
| | | | CA | 1187796 | A | 28-05-1985 |
| | | | DE | 3172028 | D | 03-10-1985 |
| | | | EP | 0049061 | A | 07-04-1982 |
| | | | ES | 505803 | D | 16-06-1983 |
| | | | ES | 8307095 | A | 16-10-1983 |
| | | | GB | 2084016 | A,B | 07-04-1982 |
| | | | GR | 75049 | A | 12-07-1984 |
| | | | HK | 17386 | A | 21-03-1986 |
| | | | IE | 51597 | B | 21-01-1987 |
| | | | JP | 1055244 | B | 22-11-1989 |
| | | | JP | 1568566 | C | 10-07-1990 |
| | | | JP | 57091921 | A | 08-06-1982 |
| | | | KE | 3581 | A | 17-01-1986 |
| | | | MX | 6558 | E | 10-07-1985 |
| | | | MY | 35386 | A | 31-12-1986 |
| | | | NZ | 198241 | A | 16-12-1983 |
| | | | PT | 73686 | A | 01-10-1981 |
| | | | ZA | 8106272 | A | 29-09-1982 |
| US 4140764 | A | 20-02-1979 | GB | 1534508 | A | 06-12-1978 |
| | | | US | 4123540 | A | 31-10-1978 |
| | | | AU | 500572 | B | 24-05-1979 |
| | | | AU | 8752175 | A | 16-06-1977 |
| | | | BE | 836652 | A | 15-06-1976 |
| | | | CA | 1064039 | A | 09-10-1979 |
| | | | CH | 619230 | A | 15-09-1980 |
| | | | DE | 2555626 | A | 01-07-1976 |
| | | | DK | 574875 | A,B, | 19-06-1976 |
| | | | ES | 443597 | A | 16-08-1977 |
| | | | FR | 2294701 | A | 16-07-1976 |
| | | | IE | 42370 | B | 30-07-1980 |
| | | | IL | 48607 | A | 25-07-1979 |
| | | | JP | 51138693 | A | 30-11-1976 |
| | | | NL | 7514545 | A | 22-06-1976 |
| | | | SE | 428564 | B | 11-07-1983 |
| | | | SE | 7514029 | A | 21-06-1976 |
| | | | ZA | 7507693 | A | 24-11-1976 |
| US 4110165 | A | 29-08-1978 | GB | 1508977 | A | 26-04-1978 |
| | | | AR | 206627 | A | 06-08-1976 |

EPO FORM P0459

For more details about this annex : see Official Journal of the European Patent Office, No. 12/82

## ANNEX TO THE EUROPEAN SEARCH REPORT
## ON EUROPEAN PATENT APPLICATION NO.

EP 01 20 1884

This annex lists the patent family members relating to the patent documents cited in the above-mentioned European search report.
The members are as contained in the European Patent Office EDP file on
The European Patent Office is in no way liable for these particulars which are merely given for the purpose of information.

15-08-2001

| Patent document cited in search report | Publication date | Patent family member(s) | Publication date |
|---|---|---|---|
| US 4110165 A | | AR 216430 A | 28-12-1979 |
| | | AR 219050 A | 31-07-1980 |
| | | AT 350718 B | 11-06-1979 |
| | | AT 205277 A | 15-11-1978 |
| | | AT 353407 B | 12-11-1979 |
| | | AT 205377 A | 15-04-1979 |
| | | AT 350719 B | 11-06-1979 |
| | | AT 205477 A | 15-11-1978 |
| | | AT 348124 B | 25-01-1979 |
| | | AT 205577 A | 15-06-1978 |
| | | AT 352270 B | 10-09-1979 |
| | | AT 298575 A | 15-02-1979 |
| | | AU 499171 B | 05-04-1979 |
| | | AU 8017775 A | 21-10-1976 |
| | | BE 827926 A | 14-10-1975 |
| | | CA 1059050 A | 24-07-1979 |
| | | CA 1055395 A | 15-05-1979 |
| | | CA 1074325 A | 25-03-1980 |
| | | CA 1076120 A | 22-04-1980 |
| | | CH 629252 A | 15-04-1982 |
| | | CH 630631 A | 30-06-1982 |
| | | CS 7502721 A | 13-02-1984 |
| | | CY 1078 A | 27-12-1980 |
| | | DD 121799 A | 20-08-1976 |
| | | DD 132297 A | 20-09-1978 |
| | | DE 2517316 A | 23-10-1975 |
| | | DE 2559410 A | 23-09-1976 |
| | | DE 2559411 A | 21-10-1976 |
| | | DE 2560074 C | 28-03-1985 |
| | | DK 167275 A,B, | 21-10-1975 |
| | | DK 286980 A,B, | 02-07-1980 |
| | | DK 287078 A,B, | 26-06-1978 |
| | | DK 287178 A | 26-06-1978 |
| | | DK 287278 A,B, | 26-06-1978 |
| | | DK 287378 A,B, | 26-06-1978 |
| | | EG 12445 A | 31-12-1978 |
| | | ES 436766 A | 16-08-1977 |
| | | FI 751133 A,B, | 21-10-1975 |
| | | FI 780201 A,B, | 23-01-1978 |
| | | FI 780202 A | 23-01-1978 |
| | | FR 2267778 A | 14-11-1975 |
| | | HK 54780 A | 03-10-1980 |
| | | HU 173700 B | 28-07-1979 |
| | | IE 41109 B | 24-10-1979 |
| | | IE 41110 B | 24-10-1979 |
| | | IL 47087 A | 25-07-1979 |

For more details about this annex : see Official Journal of the European Patent Office, No. 12/82

**ANNEX TO THE EUROPEAN SEARCH REPORT
ON EUROPEAN PATENT APPLICATION NO.**                EP 01 20 1884

This annex lists the patent family members relating to the patent documents cited in the above-mentioned European search report.
The members are as contained in the European Patent Office EDP file on
The European Patent Office is in no way liable for these particulars which are merely given for the purpose of information.

15-08-2001

| Patent document cited in search report | | Publication date | Patent family member(s) | | Publication date |
|---|---|---|---|---|---|
| US 4110165 | A | | IN | 140042 A | 04-09-1976 |
| | | | JP | 1054511 C | 23-07-1981 |
| WO 9607408 | A | 14-03-1996 | AU | 3471995 A | 27-03-1996 |
| | | | EP | 0782443 A | 09-07-1997 |
| | | | JP | 10505595 T | 02-06-1998 |
| | | | TR | 960190 A | 21-06-1996 |
| WO 9416696 | A | 04-08-1994 | AU | 5863894 A | 15-08-1994 |
| | | | DE | 69425131 D | 10-08-2000 |
| | | | DE | 69425131 T | 15-03-2001 |
| | | | EP | 1034784 A | 13-09-2000 |
| | | | EP | 0680322 A | 08-11-1995 |
| | | | JP | 8505868 T | 25-06-1996 |

EPO FORM P0459

For more details about this annex : see Official Journal of the European Patent Office, No. 12/82